# FASCICULE DE BREVET EUROPEEN

(11) **EP 2 755 940 B1**
(45) Date de publication et mention de la délivrance du brevet: **30.08.2017**
(21) Numéro de dépôt: 12759147.7
(22) Date de dépôt: 14.09.2012
(51) Int. Cl.: C07C 49/84, A61K 31/122, A61P 35/00

(54) **PRINCIPE ACTIF ANTICANCÉREUX ISSU DE GUIERA SENEGALENSIS**
AUS GUIERA SENEGALENSIS GEWONNENER WIRKSTOFF GEGEN KREBS
ANTICANCER ACTIVE INGREDIENT DERIVED FROM GUIERA SENEGALENSIS

(30) Priorité: 16.09.2011 FR 1158256
(43) Date de publication de la demande: 23.07.2014
(73) Titulaire: université Clermont Auvergne, 63000 Clermont Ferrand (FR); Ecole Nationale Supérieure de Chimie de Clermont Ferrand, ENSCCF, 63174 Aubière Cédex (FR)
(72) Inventeur: CHALARD, Pierre, F-63450 Chanonat (FR); CALDEFIE-CHEZET, Florence, F-63500 Issoire (FR); DELORT, Laetitia, F-63000 Clermont-Ferrand (FR); PATEL, Kirti, 15074 Lima (PE)
(74) Mandataire: Lavoix
(86) Numéro de dépôt international: PCT/EP2012/068125
(87) Numéro de publication internationale: WO 2013/037964

(56) Documents cités:
- FIOT J ET AL: "Phytochemical and pharmacological study of roots and leaves of Guiera senegalensis J.F. Gmel (Combretaceae)", JOURNAL OF ETHNOPHARMACOLOGY, ELSEVIER SCIENTIFIC PUBLISHERS LTD, IE, vol. 106, no. 2, 30 juin 2006 (2006-06-30) , pages 173-178, XP025085870, ISSN: 0378-8741, DOI: 10.1016/J.JEP.2005.12.030 [extrait le 2006-06-30]
- OLGA SILVA ET AL: "Guieranone A, a Naphthyl Butenone from the Leaves of Guiera senegalensis with Antifungal Activity", JOURNAL OF NATURAL PRODUCTS, vol. 66, no. 3, 1 mars 2003 (2003-03-01), pages 447-449, XP055015836, ISSN: 0163-3864, DOI: 10.1021/np0204904 cité dans la demande

## Description

La présente invention est relative à un nouveau composé utilisable à titre de principe actif pharmaceutique, notamment dans le traitement du cancer. Ce composé peut se présenter sous forme d'un extrait de plante concentré ou sous forme de composé de synthèse.

*Guiera senegalensis* est une plante très commune en Afrique tropicale. Espèce soudano sahélienne, ses zones d'abondance sont principalement en Afrique occidentale. Cette plante est très commune dans le sahel où elle forme des peuplements mono spécifiques. Elle abonde dans les jachères sur sols argileux ou sableux, est localement grégaire, et existe aussi dans les limons des vallées passagèrement inondées. Elle est présente dans presque tout le Mali, sur une zone s'étendant de Kayes à Gossi (région de Tombouctou). Espèce envahissante des friches sablonneuses, première colonisatrice des terrains brûlés par les feux de brousse dans les savanes soudaniennes et soudano-guinéennes, élément des savanes dégradées, elle est également une plante pionnière disséminée par le bétail dans les jachères, et est aussi indicateur de surpâturage. L'aire géographique du *Guiera* et ses zones d'abondance ont été principalement établies en Afrique de l'Ouest. Son aire s'étale parallèlement à l'équateur du Sénégal aux frontières de l'Ethiopie.

**Taxonomie, tableau 1 :**

| | |
|---|---|
| Règne | Végétal |
| Sous-règnes | Eucaryotes |
| Embranchement | Spermaphytes |
| Sous-embranchement | Angiospermes |
| Classe | Dicotylédones |
| Sous-classe | Rosidae |
| Ordre | Myrtales |
| Famille | Combrétacées |
| Genre | *Guiera* |
| Espèce | *senegalensis* |

On reconnaît principalement à *Guiera senegalensis* les propriétés anti-inflammatoires, antiseptiques, béchiques, diurétiques, eupnéiques et fébrifuges. D'où sa prescription pour la toux, les états dyspnéiques, le paludisme, les pneumopathies, les bronchopathies. Ce sont les feuilles qui sont généralement utilisées sous forme de décocté ou macéré en bain et comme boisson. En usage externe, *Guiera senegalensis* est considéré comme vulnéraire antiseptique, cicatrisant pour le traitement des blessures, des stomatites, gingivites, des chancres syphilitiques (Kerharo J. et Adams(1974), La Pharmacopée sénégalaise Traditionnelle. Plantes Médicinales et Toxiques. Imprimerie JOUVE, 17 rue du LOUVRE, 75001 PARIS. 354).

Un méthoxylate naphtyl butènone, appelé Guieranone A, a été isolé des feuilles du *Guiera senegaiensis.* (Olga Silva and Elsa T. Gomes. (2003), Guieranone A, a Naphtyl Butenone from the Leaves of Guiera senegalensis with Antifungal Activity. J. Nat. Prod., 66, 447).

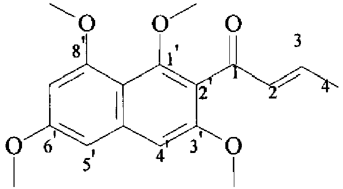

### Guieranone A

L'activité anti-cancéreuse de la Guieranone A a fait l'objet de travaux rapportés dans Fiot J. et al., J. of Ethnopharmacology, Elsevier Scientific Publisher LTD, IF, vol, 106, n° 2, 30 juin 2006, pp 173-178.
Les inventeurs ont pu isoler un nouveau composé à partir de *Guiera senegalensis.* Ce nouveau composé appelé Guieranone B a la formule (I) suivante.

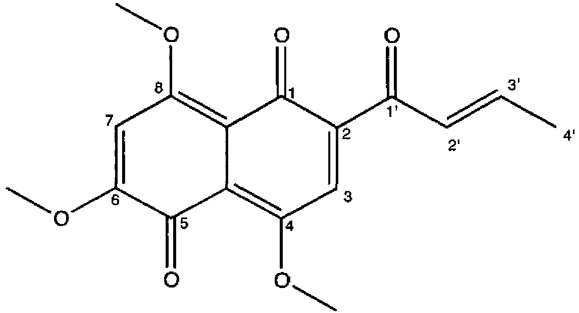

Les inventeurs ont en outre découvert que ce composé présente une activité antiproliférative élevée, comme cela a été démontré sur la lignée de cellules cancéreuses mammaires MCF-7. Le tableau 2 ci-dessous décrit l'activité antiproliférative comparée de 3 principes actifs (tamoxifène, 5-FU et Guieranone B) sur la lignée cancéreuse mammaire MCF-7 testée dans les mêmes conditions au laboratoire. La CI50 observée pour la Guieranone B (7,09 µM) est comprise entre les valeurs observées pour le tamoxifène et le 5-FU. La Guieranone B est donc un principe actif intéressant pour le traitement des cancers du sein, notamment.

**Tableau 2**

| | CI50 moyenne en µg/mL | CI50 moyenne en µM |
|---|---|---|
| Tamoxifène | 4,42 ± 0,34 | 11,91 ± 0,93 |
| 5-FU | 0,53 ± 0,05 | 4,04 ± 0,39 |
| Guieranone B | 2,24 ± 0,13 | 7,09 ± 0,41 |

Un objet de l'invention est donc de proposer la Guieranone B comme nouveau composé ou principe actif pharmaceutique, notamment comme nouvel agent anti-cancéreux.

Un autre objet de l'invention est d'isoler ce nouveau composé sous une forme substantiellement pure à partir de *Guiera senegalensis.*

Un autre objet de l'invention est d'administrer ce nouveau composé à l'homme ou à l'animal pour traiter le cancer.

La caractérisation de ce nouveau composé et la description d'un procédé d'extraction-purification permet de disposer d'extraits concentrés en ce composé. Notamment, le procédé décrit aux exemples permet d'obtenir composé pur à plus de 89 % molaire. Cette mesure a été effectuée par HPLC. Le produit obtenu peut ensuite servir à purifier davantage le composé identifié et caractérisé, pour obtenir un composé ayant un degré de pureté supérieur.

La présente invention a donc pour objet le composé de formule (I) ci-dessus ou un de ses sels d'addition avec des bases ou des acides pharmaceutiquement acceptables, sous une forme isolée ou substantiellement pure. Par substantiellement pur, on entend que le composé (I) est pur à plus de 80, 85 ou 89% molaire.

L'invention a aussi pour objet un extrait issu de *Guiera senegalensis* ou une composition concentrée comprenant au moins 50 % molaire du composé de formule (I), notamment au moins 60, 70, 80, 85 ou 89% molaire de ce composé. L'invention a notamment pour objet une composition concentrée comprenant au moins 80% molaire, de préférence au moins 85% molaire, de composé de formule (I).

Conformément à l'invention, ce composé ou cet extrait ou composition concentrée est utilisable en tant que médicament, notamment en tant que médicament anticancéreux.

L'invention a donc aussi pour objet une composition pharmaceutique ou anticancéreuse comprenant un composé de formule (I), notamment substantiellement pur, ou une composition concentrée selon l'invention, et un véhicule ou excipient pharmaceutiquement acceptable.

Elle a aussi pour objet une composition, notamment pharmaceutique, comprenant à titre de principe actif un extrait selon l'invention et un véhicule ou excipient pharmaceutiquement acceptable.

Le composé selon l'invention peut être utilisé en combinaison avec au moins un autre agent pharmaceutique, notamment agent anticancéreux. Il peut ainsi être utilisé en combinaison avec d'autres agents pharmaceutiques ou anticancéreux autorisés par les autorités réglementaires.

L'invention a donc aussi pour objet une composition comprenant le composé de formule (I), notamment substantiellement pur, ou une composition concentrée selon l'invention, et (au moins) un autre agent pharmaceutique ou agent anticancéreux, dans un véhicule ou excipient pharmaceutiquement acceptable.

L'invention a aussi pour objet une composition comprenant le composé de formule (I), notamment substantiellement pur, ou une composition concentrée selon l'invention, et (au moins) un autre agent pharmaceutique ou anticancéreux à l'exception d'un agent présent dans *Guiera senegalensis,* dans un véhicule ou excipient pharmaceutiquement acceptable.

L'invention a aussi pour objet une composition comprenant le composé de formule (I), notamment substantiellement pur, ou une composition concentrée selon l'invention, et (au moins) un autre agent pharmaceutique ou agent anticancéreux, notamment à l'exception d'un agent présent dans *Guiera senegalensis,* pour une utilisation simultanée, séparée ou étalée dans le temps du composé de formule (I) ou de la composition concentrée et de l'autre agent pharmaceutique ou anticancéreux. Ces autres agents se présentent sous forme de médicament pouvant être administré simultanément (dans une formulation commune, éventuellement extemporanée, avec le composé de formule (I)), séparément (selon un protocole de traitement prévoyant une administration concomitante des formulations séparées) ou encore étalée dans le temps (selon un protocole de traitement prévoyant une administration étalée dans le temps des formulations séparées).

Par exemple, l'autre agent est choisi parmi le tamoxifène, le 5-FU, la vinorelbine, le docetaxel ou leurs mélanges. Dans un mode de réalisation, l'autre agent anticancéreux est le tamoxifène. Dans un autre mode de réalisation, il s'agit du 5-FU. Dans un autre mode de réalisation, il s'agit de la vinorelbine. Dans un autre mode de réalisation, il s'agit du docetaxel.

Le composé, l'extrait ou les compositions concentrées selon l'invention, associés ou non à un autre agent, peuvent notamment être utilisés dans le traitement du cancer. Dans un mode de réalisation, il s'agit du cancer du sein. Dans un autre mode de réalisation, il s'agit du cancer de la prostate. Dans un autre mode de réalisation, il s'agit du cancer du colon.

L'invention a donc aussi pour objet l'utilisation du composé de formule (I), notamment substantiellement pur, ou une composition concentrée selon l'invention, pour la fabrication d'un médicament, notamment d'un médicament pour traiter le cancer. Dans un mode de réalisation, il s'agit du cancer du sein. Dans un autre mode de réalisation, il s'agit du cancer de la prostate. Dans un autre mode de réalisation, il s'agit du cancer du colon.

Les compositions selon l'invention peuvent être présentées sous toute forme appropriée à la voie d'administration prévue. La voie parentérale est la voie d'administration préférentielle et notamment la voie intraveineuse.

Les compositions selon l'invention pour administration parentérale peuvent être des solutions stériles aqueuses ou non aqueuses, des suspensions ou des émulsions. Comme solvant ou véhicule, on peut employer le propylène glycol, les huiles végétales, en particulier l'huile d'olive, et les esters organiques injectables, par exemple l'oléate d'éthyle. Ces compositions peuvent également comprendre des adjuvants en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également sous forme de compositions solides stériles qui peuvent être dissoutes ou dispersées dans de l'eau stérile ou tout autre milieu stérile injectable.

La description décrit également une méthode de traitement d'une pathologie sensible au composé de formule (1), en particulier de cancer, comprenant l'administration, au patient qui en a besoin, d'une quantité efficace du composé selon l'invention, notamment substantiellement pur, ou sous forme d'extrait ou d'une composition concentrée, avec un véhicule ou excipient pharmaceutiquement acceptable. Dans un mode de réalisation, il s'agit
du cancer du sein. Dans un autre mode de réalisation, il s'agit du cancer de la prostate. Dans un autre mode de réalisation, il s'agit du cancer du colon.

On entend par quantité suffisante, une quantité de composé selon la présente invention efficace pour limiter l'évolution de la maladie, la faire régresser ou la faire disparaître. La quantité suffisante peut être déterminée par l'homme du métier, par l'intermédiaire de technique conventionnelle et par l'observation des résultats obtenus dans des circonstances analogues. Pour déterminer la quantité suffisante différents facteurs doivent être pris en compte par l'homme du métier, notamment et sans y être limité : le sujet, sa taille, son âge, son état de santé général, la maladie impliquée et son degré de sévérité ; la réponse du sujet, le type de composé, le mode d'administration, la biodisponibilité de la composition administrée, le dosage, l'utilisation concomitante d'autres médicaments, etc.

La description décrit aussi la fraction appelée KP-18^{E} dont le mode de préparation est décrit dans les exemples. L'invention a ainsi pour objet une composition pharmaceutique ou anticancéreuse comprenant cette fraction, et ses utilisations conformes à l'invention.

L'invention va être maintenant décrite plus en détail à l'aide d'exemples pris à titre non limitatif.

### Exemple 1 : Protocole d'extraction du composé de formule (I)

L'extraction a été effectuée à partir de 1100 g de feuilles de *Guiera senegalensis* (récoltées à proximité de la ville de Ségué, région de Mopti, Mali).. Le matériel végétal renferme souvent des quantités appréciables de graisses, il a été procédé à une délipidation par agitation mécanique des feuilles broyées avec 7,5 litres de cyclohexane durant 48 heures. Le matériel végétal a ensuite été filtré sous vide et séché.

Après séchage l'extraction a été réalisée en 3 étapes:
1^{ère} Etape : Les feuilles pulvérisées, délipidées et séchées ont été placées dans un erlenmeyer contenant 3,75 litres de méthanol. 1,25 litre d'une solution aqueuse d'ammoniaque à 33% a été ajoutée. La solution possède un pH compris entre 9 et 10. L'ensemble a été mis sous agitation mécanique pendant 48 heures. Au terme des 48 heures il a été procédé à une filtration sous vide, puis le filtrat a été concentré et au filtrat a été ajouté 0,65 litre de dichlorométhane.
2^{ème} Etape : La phase organique (dichlorométhane) a été séparée du marc à l'aide d'une ampoule à décanter. Après séchage sur Na₂SO₄, elle a ensuite été concentrée partiellement à l'évaporateur rotatif jusqu'à un volume d'environ 0,4 litre. 0,3 litre d'une solution aqueuse d'acide chlorhydrique dilué à 5% a été ajouté. Ce mélange a été mis sous agitation 24 heures et la phase aqueuse a été séparée à l'aide d'une ampoule à décanter.
3^{ème} Etape : La phase aqueuse précédente a été alcalinisée par ajout d'une solution aqueuse de soude 1M jusqu'à pH=8, en présence de 0,5 litre de dichlorométhane. La phase organique (dichlorométhane) a été séparée à l'aide d'une ampoule à décanter et la phase aqueuse a été ré-extraite deux fois avec 0,2 litre de dichlorométhane. Les phases organiques (dichlorométhane) rassemblées ont été séchées sur Na₂SO₄, filtrées et évaporées sous pression réduite.

On a ainsi obtenu 3,3 g d'un extrait brut.

### Exemple 2 : Protocole de séparation de l'extrait brut :

Une séparation par chromatographie sur gel de silice a ensuite été effectuée. Cette étape permet une séparation des molécules selon leur polarité. Le résultat des tests chromatographiques a conduit à utiliser comme système d'élution un mélange Cyclohexane/Acétate d'éthyle/Méthanol en gradient de polarité.

Une masse d'environ 1 g d'extrait brut a été déposée sur une colonne de gel de silice (type 60, 230-400 mesh, Merck) préparée dans un mélange cyclohexane/acétate d'éthyle 95/5. L'élution a été réalisée par un gradient de polarité du système Cyclohexane/Acétate d'éthyle/Méthanol en commençant par un mélange cyclohexane/acétate d'éthyle 95/5 et en terminant par du méthanol pur avec un fractionnement en fonction des composants. Le suivi de ces fractions a été effectué par chromatographie sur couche mince de gel de silice sur support de verre. Les plaques ont été visualisées sous lumière UV (à 254 nm), puis révélées avec de l'acide phosphomolybdique. La progression de ces colonnes est rassemblée dans le tableau 3.

**Tableau 3: Résultats de la séparation par chromatographie sur colonne de l'extrait des alcaloïdes totaux des feuilles du site de Ségué.**

| Lot de fraction | N° de la fraction | Système d'élution | | |
|---|---|---|---|---|
| | | % cyclohexane | % acétate d'éthyle | % Méthanol |
| 01-10 | Sf1 | 95 | 5 | - |
| 11-21 | Sf2 | 95 | 5 | - |
| 22-26 | Sf3 | 90 | 10 | - |
| 27-38 | Sf4 | 90 | 10 | - |
| 39-52 | Sf5 | 90 | 10 | - |
| 53-70 | Sf6 | 90 | 10 | - |
| 71-81 | Sf7 | 90 | 10 | - |
| 82-135 | Sf8 | 85 | 15 | - |
| 139-179 | Sf9 | 85 | 15 | - |
| 180-186 | Sf10 | 80 | 20 | - |
| 187-191 | Sf11 | 80 | 20 | - |
| 192-207 | Sf12 | 80 | 20 | - |
| 208-211 | Sf13 | 80 | 20 | - |
| 212-308 | Sf14 | 75 | 25 | - |
| 309-410 | Sf15 | 75 | 25 | - |
| 411-495 | Sf16 | 65 | 35 | - |
| 496-623 | Sf16 | 55 | 45 | - |
| 624-689 | Sf17 | 40; 30 ; - | 60; 70 ; 100 | - |
| 690-696 | Sf18 | - | 90 | 10 |
| 697-734 | Sf19 | - | 80 | 20 |
| 735-755 | Sf20 | - | 50 | 50 |
| - | Sf21 | - | - | 100 |

Après rassemblement selon les résultats du suivi analytique, les fractions ont été éluées dans un système cyclohexane/acétate d'éthyle/méthanol avec différentes proportions. Ainsi il a été possible de regrouper certaines fractions et d'obtenir au final 11 fractions pour les feuilles de Ségué (tableau 4).

**Tableau 4: Regroupement des fractions issues de la colonne chromatographique de l'extrait d'alcaloïdes totaux des feuilles du site de Ségué.**

| N° des fractions | Nom de la fraction et quantité (mg) | Observations |
|---|---|---|
| Sf1-Sf3 | SF1 (38) | 1 composé révélé à l'aide de l'acide phosphomolybdique |
| Sf4 | SF2 (3) | apparemment pas de composé |
| Sf5-Sf6 | SF3 (28) | mélange de deux composés visible à l'UV |
| Sf7 | SF4 (4) | 1 composé visible à l'UV |
| Sf8-Sf13 | SF5 (218) | 3 composés visibles à l'UV |
| Sf14-Sf16 | SF6 (133) | 2 composés visibles à l'UV |
| Sf17 | SF7 (149) | 2 composés : un visible à l'UV et l'autre révélé à l'acide phosphomolybdique |
| Sf18 | SF8 (68) | 3 composés visibles à l'UV |
| Sf19 | SF9 (122) | sous forme de trainée (impuretés) |
| Sf20 | SF10 (56) | 3 composés visibles à l'UV |
| Sf21 | SF11 (500) | 2 Composés visibles à l'UV |

### Exemple 3 : Isolement et purification de la fraction KP-18E (Guieranone B ou composé de formule (I))

Une séparation chromatographique sur colonne a été ensuite effectuée sur la fraction SF5 qui présente une activité anticancéreuse intéressante. Cette étape permet une séparation plus précise des molécules selon leur polarité. Le résultat des tests chromatographiques a conduit à utiliser comme système d'élution un mélange Cyclohexane/Acétate d'éthyle/Dichlorométhane/Méthanol en gradient de polarité.

Une masse de 210 mg de SF5 a été déposée sur une colonne de gel de silice (type 60, 230-400 mesh, Merck) préparée dans du cyclohexane/acétate d'éthyle 90/10. Les dimensions de colonne de silice est 22cm hauteur et 3 cm diamètre. L'élution a été réalisée par un gradient de polarité du système Cyclohexane/Acétate d'éthyle/Méthanol en commençant par un mélange cyclohexane/acétate d'éthyle 90/10 et en terminant par du dichlorométhane/méthanol (0,2% triéthylamine) avec un fractionnement en fonction des composants. Le suivi de ces fractions a été effectué par chromatographie sur couche mince de gel de silice sur support de verre. Les plaques ont été visualisées sous lumière UV (à 254 nm), puis révélées avec de l'acide phosphomolybdique. La progression de ces colonnes a été rassemblée dans le tableau 3. Après rassemblement selon les résultats du suivi analytique, 9 fractions différentes ont été obtenues.

Parmi celles-ci, la fraction KP-18E, représentant 17 mg, est constituée du composé de l'invention, qui a été dénommé Guieranone B.

**Tableau 5: Regroupement des fractions issues de la colonne chromatographique de la fraction SF5**

| Système d'élution (numéro de tubes) | Lot de fraction | Nom de la fraction et quantité (mg) | Observations |
|---|---|---|---|
| Cyclohex 9 /EtOAc 1 (1-35) | 1-29 | KP-18A (20) | 2 taches visibles en UV jaune dans silice |
| | 30-35 | KP-18B (100) | 1 tache visible en UV et 2 taches révélées à l'acide phosphomolybdique jaune dans silice |
| Cyclohex 8 /EtOAc 2 (36-52) | 36-56 | KP-18C (21) | 3 tache visibles en UV rose dans silice |
| Cyclohex 7 /EtOAc 3 (53-83) | 57-69 | KP-18D (10) | 1 tache visible en UV rose dans silice |
| Cyclohex 6 /EtOAc 4 (84-104) | 70- 86 | KP-18E (17) | 1 tache visible en UV rose dans silice |
| Cyclohex 5 /EtOAc 5 (105-122) | 87-140 | KP-18F (9) | Trace visible en UV rose dans silice |
| Cyclohex 4 /EtOAc 6 (123-140) | | | |
| Cyclohex 3 /EtOAc 7 (100 ml) | | KP-18G (8) | 1 tache visible en UV rose dans silice |
| Cyclohex 2 /EtOAc 8 (150 ml) | | KP-18H (5) | Trace visible en UV rose dans silice |
| Cyclohex 1 /EtOAc 9 | | KP-18I | Trace visible en UV |
| EtOAc 100 % | | (8) | rose dans silice |
| EtOAc 9 /MeOH 1 | | | |
| DCM 8 /MeOH (0,2 % TEA) 2 | | | |
| (100ml pour chaque) | | | |

La pureté en Guieranone B dans les fractions SF5 et KP-18E a été mesurée par HPLC sur colonne Symmetry C18 4.6 x 150 mm, diam : 5mm
phase mobile : gradient [H2O + acide trifluoroacétique (0.1 %)] et MeOH

**Tableau 6 :**

| temps (min) | H2O + acide trifluoroacétique (0.1%) | MeOH |
|---|---|---|
| 0 | 95% | 5% |
| 1 | 95% | 5% |
| 45 | 5% | 95% |
| 60 | 95% | 5% |

débit : 1 mL / min
détecteur : UV 254 nm
volume injecté : 20mL

### Résultats :

- SF5 : pic caractéristique à 24,062 : aire sous la courbe 4,3681 % molaire
- KP-18E : pic caractéristique à 24,335 : aire sous la courbe 89,1262% molaire

On démontre l'obtention d'un composé de formule (I) substantiellement pur, avec une pureté molaire de plus de 89%.

### Exemple 4 : Caractérisation complète de KP-SF5-18^{E} (KP-18E)

Guieranone B (C₁₇H₁₆0₆ - 316 g.mol ⁻¹). TOF (pos.) *m*/*z* 339 [M+Na]⁺, HRMS-TOF (pos) m/z 317,10181, ([M+H]⁺, calcd. pour C₁₇H₁₇0₆, 317, 10150).

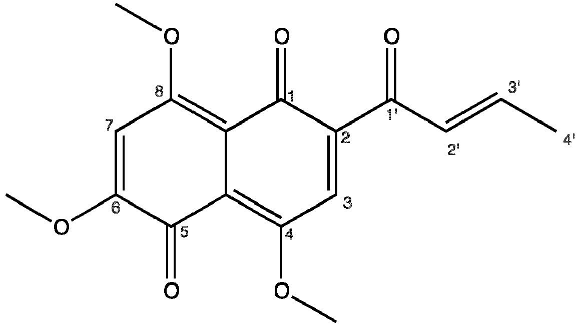

13C RMN (CDCl₃-*d1*, 100 MHz) δ 18,0 (C4'); 56,0 (OCH₃-4, OCH₃-8); 63,2 (OCH₃-6); 105,5 (C3); 111,9 (C7); 117,3 (C8a); 130,7 (C2); 134,2 (C2'); 134,9 (C4a); 148,9 (C3'); 158,5 (C6); 158,7 (C8); 159,3 (C4); 179,2 (C1); 182,8 (C5); 193,1 (C1').

¹H RMN (CDCl₃-*d1*, 400 MHz) δ 7,56 (s, 1H, H3); 6,59 (m, 1H, H3'); 6,30 (d, *J* = 15,5, 1H, H2'); 6,08 (s, 1H, H7); 3,95 (s, 3H, O-CH₃-4); 3,90 (s, 3H, O-CH₃-4); 3,82 (s, 3H, O-CH₃-6); 1,94 (d, *J* = 6,60 Hz, 3H, H4')

**Tableau 7:**

| Position | H | C | HMBC |
|---|---|---|---|
| 1 | | 179,2 | |
| 2 | | 130,7 | |
| 3 | 7,56, s, 1H | 105,5 | 1, 2, 4, 4a, 8a, 1', 2' |
| 4 | | 159,3 | |
| OCH₃-4 | 3,95, s, 3H | 56,0 | 4 |
| 4a | | 134,9 | |
| 5 | | 182,8 | |
| 6 | | 158,5 | |
| OCH₃-6 | 3,82, s, 3H | 63,2 | 6 |
| 7 | 6,08, s, 1H | 111,9 | 1, 5, 6, 8, 8a |
| 8 | | 158,7 | |
| OCH₃-8 | 3,90, s, 3H | 56,0 | 7,8 |
| 8a | | 117,3 | |
| 1' | | 193,1 | |
| 2' | 6,30, d, 1H (*J* = 15,5 Hz) | 134,2 | 1', 3', 4' |
| 3' | 6,59, m, 1H | 148,9 | 1', 2' 4' |
| 4' | 1,94, d, 3H (*J* = 6,60 Hz) | 18,0 | 1', 2', 3' |

Les spectres RMN ¹H, COSY (Correlation Spectroscopy), ¹³C et HMBC (¹H/¹³C) mettent en évidence la présence de deux groupements aromatiques et d'une cétone éthylénique. Les analyses des spectres de RMN ¹H, COSY, ¹³C, HSQC et HMBC (¹H/¹³C) permettent de confirmer la structure finale de KP18E (naphtylméthoxylate de buténone).

### Exemple 5 : Protocole détaillé de la mesure de prolifération des cellules cancéreuses mammaires MCF-7

### 1 ^{ère} partie Jour 1 : ensemencement des cellules

### 1. Préparation du milieu :

### -Préparation du volume pour mettre dans les plaques :

Dans un Falcon de 50 mL préparer 30 mL de RPMI complet avec 10% de SVF et 1% de Glutamine : 27 mL de RPMI ; 3 mL de SVF et 300 µL de Glutamine.

### 2. Préparation de la suspension de cellules :

- Aspirer le milieu contenu dans le flasque avec la trompe à vide
- Rincer avec 3 mL de PBS et aspirer avec la trompe à vide
- Ajouter 1 mL de trypsine et laisser agir 3 min à l'étuve à 37°C en vue de décoller les cellules.
- Lorsque les cellules sont décollées, ajouter 2 ml de milieu complet préparé précédemment pour inactiver la trypsine.
- Bien mélanger la suspension par aspiration-refoulement et transférer dans un Falcon 15 bien identifié.
- Dénombrement des cellules en cellule de Malassez : effectuer une dilution au 20^{ème} pour les compter au bleu Trypan.

### 3. Ensemencement des plaques:

- Ensemencer les plaques avec 200 µL de suspension de cellules selon le schéma déterminé
- Mettre 200 µL d'eau dans les puits périphériques
- Laisser incuber 24 h.

### 2^{ème} partie : Jour 2, mise en présence de l'extrait

### 4. Préparation du milieu:

45 mL au total de milieu à préparer dans un Falcon de 50 stérilisées

Soit 40,5 mL de RPMI + 4,5 mL de SVF + 450 µL de glutamine

### 5. Préparation des extraits :

- préparer une solution de 10 mg/mL :
   Dissoudre 8,1 mg d'extrait brut (noté SF) selon l'exemple 1, de la fraction SF5 selon l'exemple 2 ou de la fraction KP-18^{E} (Guieranone B) dans 810 µL de DMSO
- Préparation de la solution mère d'extrait (Ex) à 100 µg/mL soit 50µL de (10 mg/ml) dans 4950 µL de RPMI
- Réalisation de la gamme d'extrait (pour 1 extrait et 1 seule gamme).
- Enlever le milieu dans les plaques et ajouter 200 µL par puits d'une gamme d'extrait (une colonne par concentration).
- Mettre à incubation à l'étuve les plaques sous 37°C durant 72H.

### 6. Préparation de la résazurine (révélateur) et lecture de la plaque To

### • Pour T₀ :

2 colonnes de cellules + une colonne de Résazurine seule= 3 colonnes
200 µL/puits X 3 colonnes X 6 lignes= 3600 µL (volume total à préparer : 5 mL)
- Préparer 4,9 mL de milieu + 100 µL Résazurine
   Enlever le milieu et ajouter 200 µL de (milieu + Résazurine) par puits.
- Incuber pendant 2 heures, et lecture au fluoroskan.

### 3^{ème} partie : Mesure de la prolifération à 24 h (pour t₀), 72 h (pour extrait)

### • Pour les extraits à 72h :

200 µL /puits X 18 colonnes X 6 lignes (volume total à préparer : 25 mL)
- Préparer 24,5 mL de RPMI + 500 µL de Résazurine
- Enlever le milieu et ajouter 200 µL de (milieu + Résazurine) par puits.
- Au bout de 2 heures, lecture au fluoroskan.

### Résultats :

1 : Mesure de l'activité antiproliférative de SF en % d'inhibition (Criblage 0 à 100 µg/mL)

**Tableau 8**

| SF | Pourcentage d'inhibition | | | | |
|---|---|---|---|---|---|
| Concentrations | 0 µg/mL | 12,5µg/mL | 25 µg/mL | 50 µg/mL | 100 µg/mL |
| Valeurs | 0,00 | 98,10 | 96,79 | 100,00 | 100,00 |
| | 11,25 | 98,46 | 97,74 | 100,00 | 100,00 |
| | 19,60 | 98,78 | 97,57 | 100,00 | 100,00 |
| | 0,00 | 98,20 | 98,42 | 100,00 | 100,00 |
| | 5,93 | 97,07 | 97,82 | 100,00 | 100,00 |
| | 5,72 | 98,40 | 96,71 | 100,00 | 100,00 |
| Moyennes ESM | 7,08 | 98,17 | 97,51 | 100,00 | 100,00 |
| | 3,04 | 0,24 | 0,27 | 0,00 | 0,00 |

2 : Mesure de l'activité antiproliférative de SF en % d'inhibition (Criblage 0 à 10 µg/mL)

**Tableau 9**

| SF | Pourcentage d'inhibition | | | | | | | |
|---|---|---|---|---|---|---|---|---|
| Concentrations | 0 µg/mL | 0,5 µg/mL | 1 µg/mL | 2 µg/mL | 4 µg/mL | 6 µg/mL | 8 µg/mL | 10 µg/mL |
| Valeurs | 0,00 | 0,95 | 5,83 | 28,14 | 57,42 | 78,82 | 96,93 | 96,63 |
| | 0,00 | 10,85 | 26,32 | 40,97 | 73,14 | 87,61 | 98,56 | 97,98 |
| | 13,68 | 28,37 | 30,18 | 45,99 | 76,24 | 91,83 | 98,86 | 97,89 |
| Moyennes ESM | 4,56 | 13,39 | 20,78 | 38,36 | 68,94 | 86,09 | 98,12 | 97,50 |
| | 4,56 | 8,02 | 7,56 | 5,31 | 5,83 | 3,83 | 0,60 | 0,44 |

3 : Mesure de l'activité antiproliférative de SF en % d'inhibition
(Moyennes obtenues pour les 6 essais) Tableau 10

| SF | | | 0 µg/mL | 0,5 µg/mL | 1 µg/mL | 2 µg/mL | 3 µg/mL | 4 µg/mL | 6 µg/mL | 8 µg/mL |
|---|---|---|---|---|---|---|---|---|---|---|
| 1 | N1 | Moyennes | 1,87 | 0,00 | 0,00 | 9,54 | 32,14 | 58,48 | 85,92 | 97,97 |
| | N2 | Moyennes | 1,81 | 0,00 | 2,25 | 9,19 | 14,19 | 34,87 | 67,82 | 96,44 |
| | N3 | Moyennes | 1,43 | 0,00 | 0,00 | 6,34 | 17,06 | 42,39 | 74,72 | 97,54 |
| 2 | N1 | Moyennes | 0,74 | 4,73 | 5,34 | 16,93 | 35,35 | 56,34 | 81,25 | 95,94 |
| | N2 | Moyennes | 0,89 | 3,68 | 2,74 | 21,04 | 43,86 | 62,87 | 85,48 | 96,05 |
| | N3 | Moyennes | 1,50 | 3,20 | 1,13 | 8,84 | 13,44 | 36,69 | 62,80 | 94,15 |
| Moyenne des manipulations | | | 1,37 | 1,93 | 1,91 | 11,98 | 26,00 | 48,60 | 76,33 | 96,34 |
| ESM | | | 0,19 | 0,88 | 0,82 | 2,32 | 5,23 | 4,93 | 3,90 | 0,55 |
| | | | | | | | | | | |

| | | | | | | CI50 | | | | |
|---|---|---|---|---|---|---|---|---|---|---|
| | | 1 | | N1 | | 3,64 | | µg/mL | | |
| | | | | N2 | | 4,81 | | | | |
| | | | | N3 | | 4,40 | | | | |
| | | 2 | | N1 | | 3,66 | | | | |
| | | | | N2 | | 3,29 | | | | |
| | | | | N3 | | 4,91 | | | | |
| | | CI50 moyenne ± ESM | | | | 4,12 ± 0,27 | | µg/mL | | |

4: Pourcentage d'inhibition de la prolifération des cellules cancéreuses MCF-7 en fonction des concentrations de SF5 du criblage Tableau 11

| SF5 | Pourcentage d'inhibition | | | | | | | |
|---|---|---|---|---|---|---|---|---|
| Concentrations | 0 µg/mL | 0,5 µg/mL | 1 µg/mL | 2 µg/mL | 3 µg/mL | 4 µg/mL | 6 µg/mL | 8 µg/mL |
| Valeurs | 3,55 | 0 | 10,52 | 32,23 | 54,69 | 62,03 | 72,34 | 92,45 |
| | 0 | 0 | 3,52 | 27,79 | 52,88 | 58,91 | 73,28 | 94,76 |
| | 2,24 | 0 | 5,96 | 35,51 | 57,75 | 63,19 | 75,16 | 92,31 |
| | 0 | 0 | 2,27 | 33,29 | 52,72 | 59,78 | 68,72 | 90,60 |
| | 1,58 | 0 | 0 | 34,57 | 56,41 | 60,19 | 72,65 | 89,37 |
| | 1,30 | 0 | 3,46 | 31,26 | 55,16 | 60,06 | 73,06 | 87,83 |
| Moyennes ESM | 1,44 | 0 | 4,29 | 32,44 | 54,94 | 60,69 | 72,53 | 91,22 |
| | 0,55 | 0 | 1,47 | 1,12 | 0,80 | 0,65 | 0,86 | 1,00 |

5 : Mesure de l'activité antiproliférative des fractions de SF : SF5 en % d'inhibition
(Moyennes obtenues pour les 6 essais) Tableau 12

| SF5 | | | 0 µg/mL | 0,5 µg/mL | 1 µg/mL | 2 µg/mL | 3 µg/mL | 4 µg/mL | 6 µg/m L | 8 µg/mL |
|---|---|---|---|---|---|---|---|---|---|---|
| 1 | N1 | Moyennes | 2,47 | 1,02 | 26,74 | 74,39 | 84,77 | 86,41 | 89,95 | 94,46 |
| | N2 | Moyennes | 1,22 | 0,71 | 21,56 | 72,14 | 82,94 | 86,30 | 90,50 | 94,89 |
| | N3 | Moyennes | 1,62 | 6,45 | 17,76 | 70,17 | 82,82 | 86,00 | 90,15 | 94,31 |
| 2 | N1 | Moyennes | 3,14 | 18,51 | 55,12 | 84,52 | 92,33 | 95,50 | 99,30 | 100,00 |
| | N2 | Moyennes | 1,76 | 19,97 | 47,68 | 79,40 | 89,05 | 94,02 | 97,60 | 100,00 |
| | N3 | Moyennes | 2,07 | 20,15 | 50,33 | 82,44 | 90,30 | 92,81 | 95,08 | 98,87 |
| Moyenne des manips | | | 2,04 | 11,13 | 36,53 | 77,17 | 87,03 | 90,17 | 93,76 | 97,09 |
| ESM | | | 0,27 | 3,85 | 6,66 | 2,37 | 1,65 | 1,79 | 1,68 | 1,14 |
| | | | | | | | | | | |
| | | | | | | CI50 SF5 | | | | |
| | | | 1 | N1 | | 1,40 | | µg/mL | | |
| | | | | N2 | | 1,47 | | | | |
| | | | | N3 | | 1,53 | | | | |
| | | | 2 | N1 | | 0,90 | | | | |
| | | | | N2 | | 1,05 | | | | |
| | | | | N3 | | 0,99 | | | | |
| | | | CI50 moyenne ± ESM | | | 1,22 ± 0,11 | | µg/mL | | |

6 : Pourcentage d'inhibition de la prolifération des cellules cancéreuses MCF-7 en fonction des concentrations de KP-18E du criblage Tableau 13

| KP-18E | Pourcentage d'inhibition | | | | | | | | | |
|---|---|---|---|---|---|---|---|---|---|---|
| Concentration s | 0 µg/mL | 0,25 µg/mL | 0,5 µg/mL | 1 µg/mL | 1,5 µg/mL | 2 µg/mL | 2,5 µg/mL | 3 µg/mL | 4 µg/mL | 5 µg/mL |
| Valeurs | 30,82 | 0,00 | 1,98 | 15,52 | 36,10 | 62,54 | 57,84 | 77,40 | 99,38 | 100,00 |
| | 0,00 | 0,00 | 0,00 | 0,00 | 48,15 | 43,35 | 59,61 | 86,16 | 100,00 | 100,00 |
| | 0,00 | 0,00 | 7,74 | 29,28 | 50,66 | 59,58 | 65,92 | 83,19 | 100,00 | 100,00 |
| | 22,56 | 0,00 | 22,99 | 27,15 | 53,59 | 61,13 | 65,73 | 84,81 | 100,00 | 100,00 |
| | 0,00 | 0,00 | 1,87 | 39,25 | 53,16 | 48,79 | 68,02 | 92,10 | 100,00 | 100,00 |
| | 3,37 | 25,60 | 31,52 | 37,44 | 49,86 | 68,59 | 69,73 | 94,22 | 100,00 | 100,00 |
| Moyennes ESM | 9,46 | 4,27 | 11,02 | 24,77 | 48,59 | 57,33 | 64,48 | 86,31 | 99,90 | 100,00 |
| | 5,58 | 4,27 | 5,36 | 6,04 | 2,63 | 3,84 | 1,93 | 2,50 | 0,10 | 0,00 |

7 : Mesure de l'activité antiproliférative de KP-18E en % d'inhibition (Moyennes obtenues pour les 6 essais) Tableau 14

| µg/mL | | | 0 | 0,25 | 0,5 | 1 | 1,5 | 2 | 2,5 | 3 | 4 | 5 |
|---|---|---|---|---|---|---|---|---|---|---|---|---|
| 1 | N1 | Moyennes | 3,23 | 0,89 | 4,08 | 16,32 | 33,96 | 46,74 | 74,28 | 95,88 | 99,55 | 99,43 |
| | N2 | Moyennes | 1,95 | 0,00 | 0,30 | 6,50 | 8,86 | 23,88 | 40,33 | 61,22 | 98,27 | 99,91 |
| | N3 | Moyennes | 3,22 | 0,53 | 2,19 | 2,87 | 22,68 | 41,08 | 65,99 | 93,83 | 99,46 | 99,29 |
| 2 | N1 | Moyennes | 1,79 | 1,75 | 3,42 | 15,89 | 20,44 | 38,75 | 49,01 | 57,82 | 91,14 | 99,02 |
| | N2 | Moyennes | 1,90 | 4,00 | 7,57 | 20,89 | 32,06 | 48,73 | 57,31 | 70,40 | 99,55 | 100,00 |
| | N3 | Moyennes | 3,78 | 7,53 | 10,24 | 28,50 | 40,50 | 51,63 | 61,79 | 75,79 | 99,83 | 100,00 |
| Moyenne des manipulations | | | 2,65 | 2,45 | 4,63 | 15,16 | 26,41 | 41,80 | 58,12 | 75,83 | 97,96 | 99,61 |
| ESM | | | 0,35 | 1,17 | 1,49 | 3,83 | 4,64 | 4,08 | 4,96 | 6,57 | 1,38 | 0,17 |
| | | | | | | | CI50 | | | | | |
| | | | | 1 | N1 | | 2,0535 | | µg/mL | | | |
| | | | | | N2 | | 2,7202 | | | | | |
| | | | | | N3 | | 2,1664 | | | | | |
| | | | | 2 | N1 | | 2,5518 | | µg/mL | | | |
| | | | | | N2 | | 2,067 | | | | | |
| | | | | | N3 | | 1,9176 | | | | | |
| | | | | CI50 moyenne | | | 2,246083333 | | µg/mL | | | |
| | | | | ESM | | | 0,1293 | | µg/mL | | | |

### Exemple 6 : Comparaison de l'activité antiproliférative de la Guieranone B avec celle du tamoxifène et du 5-FU:

Le protocole décrit à l'exemple 5 a été utilisé, en remplaçant la Guieranone B par le tamoxifène ou le 5-FU (5-Fluorouracile). En suivant ce protocole, il a été possible de mesurer les CI50 de ces composés et de les comparer à celle de la Guieranone B.

Le tableau 15 (identique au tableau 2) décrit l'activité antiproliférative comparée de ces 3 principes actifs (tamoxifène, 5-FU et Guieranone B) sur la lignée cancéreuse mammaire MCF-7 testée dans les mêmes conditions.

| | CI50 moyenne en µg/mL | CI50 moyenne en µM |
|---|---|---|
| Tamoxifène | 4,42 ± 0,34 | 11,91 ± 0,93 |
| 5-FU | 0,53 ± 0,05 | 4,04 ± 0,39 |
| Guieranone B | 2,24 ± 0,13 | 7,09 ± 0,41 |

La CI50 observée pour la Guieranone B (7,09 µM) est comprise entre les valeurs observées pour le tamoxifène et le 5-FU. La Guieranone B est donc un principe actif potentiellement intéressant pour le traitement des cancers du sein.

## Revendications

1. Composé substantiellement pur de formule (I) 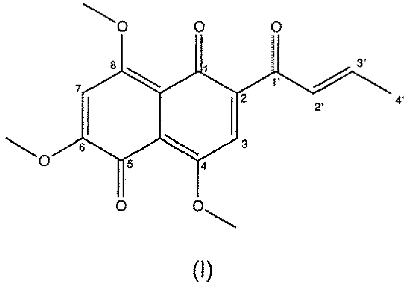 ou un de ses sels d'addition avec des bases ou des acides pharmaceutiquement acceptables.

2. Composition concentrée comprenant au moins 80% molaire, de préférence au moins 85% molaire, de composé de formule (I) selon la revendication 1.

3. Composition pharmaceutique ou anticancéreuse comprenant un composé selon la revendication 1 ou une composition concentrée selon la revendication 2 et au moins un véhicule ou excipient pharmaceutiquement acceptable.

4. Composition pharmaceutique ou anticancéreuse selon la revendication 3, comprenant au moins un autre agent pharmaceutique ou anticancéreux.

5. Composition pharmaceutique ou anticancéreuse selon la revendication 4, pour une utilisation simultanée, séparée ou étalée dans le temps du composé de formule (I) ou de la composition concentrée selon la revendication 2 et de l'autre agent pharmaceutique ou anticancéreux.

6. Composition pharmaceutique ou anticancéreuse selon les revendications 3 ou 4 dans laquelle l'autre agent est choisi parmi le tamoxifène, le 5-FU, la vinorelbine, le docetaxel ou leurs mélanges.

7. Composé selon la revendication 1, composition concentrée selon la revendication 2 ou composition pharmaceutique ou anticancéreuse selon les revendications 3 à 6 en tant que médicament, de préférence en tant que médicament anticancéreux.

8. Composé selon la revendication 1, composition concentrée selon la revendication 2 ou composition pharmaceutique ou anticancéreuse selon les revendications 3 à 6, pour son utilisation dans le traitement du cancer, de préférence pour le traitement du cancer du sein, du colon ou de la prostate.

9. Extrait concentré de *Guiera senegalensis* comprenant plus de 50% molaire de composé de la revendication 1, en tant que médicament anticancéreux, de préférence pour le traitement du cancer du sein, du colon ou de la prostate.

## Patentansprüche

1. Im Wesentlichen reine Verbindung der Formel (I) 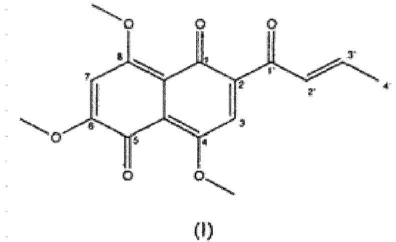 oder eines ihrer Additionssalze mit Basen oder Säuren, die pharmazeutisch verträglich sind.

2. Konzentrierte Zusammensetzung, mindestens 80 Mol-%, vorzugsweise mindestens 85 Mol-%, der Verbindung der Formel (I) nach dem Anspruch 1.

3. Pharmazeutische Zusammensetzung oder Antikrebs-Zusammensetzung, eine Verbindung nach Anspruch 1 oder eine konzentrierte Zusammensetzung nach Anspruch 2 und mindestens ein pharmazeutisch verträgliches Vehikel oder Trägerstoff enthaltend.

4. Pharmazeutische Zusammensetzung oder Antikrebs-Zusammensetzung nach Anspruch 3, mindestens ein anderes pharmazeutisches Agens oder Antikrebs-Agens enthaltend.

5. Pharmazeutische Zusammensetzung oder Antikrebs-Zusammensetzung nach Anspruch 4 zur gleichzeitigen, getrennten oder über die Zeit verteilten Verwendung der Verbindung der Formel (I) oder der konzentrierten Zusammensetzung nach Anspruch 2 und des anderen pharmazeutischen Agens oder Antikrebs-Agens.

6. Pharmazeutische Zusammensetzung oder Antikrebs-Zusammensetzung nach den Ansprüchen 3 oder 4, bei der das andere Agens ausgewählt ist aus Tamoxifen, 5-FU, Vinorelbin, Docetaxel oder ihren Mischungen.

7. Verbindung nach Anspruch 1, konzentrierte Zusammensetzung nach Anspruch 2 oder pharmazeutische Zusammensetzung oder Antikrebs-Zusammensetzung nach den Ansprüchen 3 bis 6 als Medikament, vorzugsweise als Antikrebsmedikament.

8. Verbindung nach Anspruch 1, konzentrierte Zusammensetzung nach Anspruch 2 oder pharmazeutische Zusammensetzung oder Antikrebs-Zusammensetzung nach den Ansprüchen 3 bis 6 zur Verwendung in der Behandlung von Krebs, vorzugsweise für die Behandlung von Brustkrebs, Darmkrebs oder Prostatakrebs.

9. Konzentrierter Extrakt von *Guiera senegalensis,* mehr als 50 Mol-% der Verbindung nach Anspruch 1 enthaltend, als Antikrebsmedikament, vorzugsweise zur Behandlung von Brustkrebs, Darmkrebs oder Prostatakrebs.

## Claims

1. A substantially pure compound of formula (I) 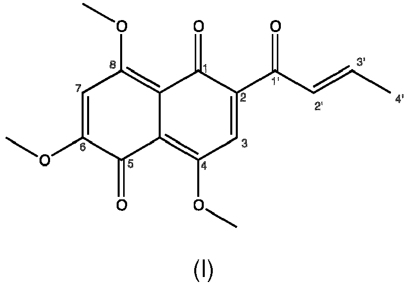 or one of its addition salts with pharmaceutically acceptable bases or acids.

2. A concentrated composition comprising at least 80% molar, preferably at least 85% molar, of the compound of formula (I) according to claim 1.

3. A pharmaceutical or anticancer composition comprising a compound according to claim 1 or a concentrated composition according to claim 2 and at least one pharmaceutically acceptable carrier or excipient.

4. The pharmaceutical or anticancer composition according to claim 3, comprising at least one other pharmaceutical or anticancer agent.

5. The pharmaceutical or anticancer composition according to claim 4, for simultaneous, separate use or spread out over time of the compound of formula (I) or of the concentrated composition according to claim 2 and of the other pharmaceutical or anticancer agent.

6. The pharmaceutical or anticancer composition according to claims 3 or 4 wherein the other agent is selected from tamoxifene, 5-FU, vinorelbin, docetaxel or mixtures thereof.

7. The compound according to claim 1, the concentrated composition according to claim 2 or the pharmaceutical or anticancer composition according to claims 3 to 6 as a drug, preferably as an anticancer drug.

8. The compound according to claim 1, the concentrated composition according to claim 2 or the pharmaceutical or anticancer composition according to claims 3 to 6, for its use in the treatment of cancer, preferably for treating breast cancer, colorectal cancer or prostate cancer.

9. A concentrated extract of *Guiera senegalensis* comprising more than 50% molar of the compound of claim 1, as an anticancer drug, preferably for treating breast cancer, colorectal cancer or prostate cancer.
